# EUROPEAN PATENT APPLICATION

(11) **EP 3 133 571 A1**
(43) Date of publication of application: **22.02.2017**
(21) Application number: 14861102.3
(22) Date of filing: 29.08.2014
(51) Int. Cl.: G08B 21/04, A61B 5/0205

(54) **EARLY-WARNING DEVICE AND EARLY-WARNING METHOD**

(30) Priority: 14.04.2014 CN 201410149049
(71) Applicant: BOE Technology Group Co., Ltd., Beijing 100015 (CN)
(72) Inventor: MU, Xinxin, Beijing 100176 (CN)
(74) Representative: Michalski Hüttermann & Partner Patentanwälte mbB
(86) International application number: PCT/CN2014/085478
(87) International publication number: WO 2015/158095

(57) **Abstract**

The present invention provides a warning device and a warning method. The warning device comprises an initial unit and a terminal unit, wherein the initial unit carried on an organism is configured to acquire a physiological state parameter of the organism and transmit warning information according to the physiological state parameter; the terminal unit is configured to receive the warning information, and notify a guardian of the warning information. As the warning device is provided with the initial unit and the terminal unit, when an physical abnormal condition such as heart attack, hypertension attack, loss of consciousness after sudden falling down occurs on the organism carrying the initial unit, the warning information can be timely sent to the terminal unit which may timely notify the organism's guardian to deal with the physical abnormal condition in time, so as not to cause delay in treatment.

## Description

### Field of the Invention

The present invention provides a warning device and a warning method.

### Background of the Invention

With increasing popularity of portable medical devices, there has been developed portable medical devices that are wearable and capable of performing real-time statistics and observation on physical conditions and health indicators. Especially, some elderly people generally carry a medical device capable of monitoring a body indicator (such as blood pressure, pulse, heartbeat, etc.) to facilitate monitoring of their physical conditions at any time.

However, it is not satisfactory that such portable medical devices have no communication function in spite of the function of monitoring physical health conditions in real time. Nevertheless, elderly people are usually unwilling to carry a cumbersome communication terminal device (such as mobile phone, pager, etc.) when going out for activities. On the one hand, carrying a communication device is inconvenient for outdoor activities; on the other hand, a communication device is complicated to operate for elderly people, for example, who may forget a phone number and it will take great effort for whom to find the phone number on the communication device, etc.

Just for this reason, in the case of abnormal physical conditions happened to the elderly people or some special groups (such as hypertension group) during outdoor activities, their friends or family members may not be informed in time because communication devices are not carried. Or, in the case of severely bad physical conditions, even faint or coma happened, their friends or family members or hospital emergency centers may not be informed timely even if communication terminals are carried, so that their friends or family members or hospital emergency centers cannot deal with the emergency in time, which often results in missing best time for first aid, causing delay in treatment of diseases of the patients, or even leading to irrecoverable bodily hurt to the patients.

### Summary of the Invention

In view of the above technical problems existing in the prior art, the present invention provides a warning device and a warning method. As the warning device is provided with an initial unit and a terminal unit, when an physical abnormal condition occurs on the organism carrying the initial unit, warning information can be timely sent to the terminal unit, so that the terminal unit timely notifies the organism's guardian to deal with the physical abnormal condition of the organism in time so as not to cause delay in treatment.

The present invention provides a warning device, including an initial unit and a terminal unit, wherein the initial unit is capable of being carried on an organism, and configured to acquire a physiological state parameter of the organism and transmit warning information according to the physiological state parameter; and the terminal unit is configured to receive the warning information, and notify the guardian of the warning information.

Preferably, the initial unit includes an acquisition module, a first processing module, a judgment module and a transmitting module. The acquisition module is configured to acquire the physiological state parameter of the organism, and transmit the physiological state parameter to the first processing module; the first processing module is configured to perform signal conversion processing on the physiological state parameter, and transmit the processed physiological state parameter to the judgment module; the judgment module is configured to judge whether a value of the processed physiological state parameter exceeds a set value, and transmit a judgment result to the transmitting module; and the transmitting module is configured to transmit the warning information to the terminal unit according to the judgment result.

Preferably, the terminal unit includes a receiving module, a second processing module and a notifying module. The receiving module is configured to receive the warning information, and transmit the warning information to the second processing module; the second processing module is configured to perform signal conversion processing on the warning information, and transmit the processed warning information to the notifying module; and the notifying module is configured to notify the guardian of the warning information.

Preferably, the initial unit further includes a first storage module and a first control module that are connected to each other. The first storage module is further connected with the acquisition module and the first processing module respectively, and configured to store the physiological state parameter acquired by the acquisition module and the processed physiological state parameter processed by the first processing module, under the control of the first control module.

The first control module is further connected with the judgment module and the transmitting module respectively, and configured to control on and off of the transmitting module according to the judgment result of the judgment module.

The first control module may further control that the physiological state parameter stored in the first storage module is read out to the transmitting module, which in turn transmits the physiological state parameter of the organism to the terminal unit.

Preferably, the terminal unit further includes a second storage module and a second control module that are connected to each other. The second storage module is further connected with the receiving module and the second processing module respectively, and configured to store the warning information received by the receiving module and the processed warning information processed by the second processing module, under the control of the second control module.

The receiving module of the terminal unit is further configured to receive the physiological state parameter of the organism from the transmitting module of the initial unit, and transmit the physiological state parameter to the second processing module; the second processing module is further configured to perform signal conversion processing on the physiological state parameter, and transmit the processed physiological state parameter to the notifying module; and the notifying module is further configured to notify the guardian of the physiological state parameter.

Preferably, the initial unit further includes a GPS positioning module. The GPS positioning module is connected with the transmitting module and the first control module, and configured to locate the geographic position of the organism; the first control module is configured to control the GPS positioning module transmitting the geographic position to the transmitting module according to the judgment result of the judgment module; and the transmitting module is further configured to transmit the geographic position to the terminal unit.

Preferably, the initial unit further includes an input module; the input module is connected with the first control module, and configured to receive a manually input control signal so as to manually control the on and off of the acquisition module and manually control the on and off of the transmitting module.

Preferably, the input module includes a microphone, a tap sensing key, a touch screen and/or a key; and the notifying module includes a speaker, a vibrator and/or a display.

Preferably, the acquisition module includes a blood pressure sensor, a pulse sensor, a heart rate sensor, an acceleration sensor and/or a gyroscope.

Preferably, the transmitting module and the receiving module are matched with each other in transmission mode or communication mode.

Preferably, the warning device further includes a ring band; the initial unit is arranged on the ring band, and the ring band is wearable on the wrist, ankle or neck of the organism; and the terminal unit is capable of being carried by the guardian or arranged at a fixed position.

The present invention also provides a warning method, including: transmitting warning information according to a physiological state parameter of an organism; and receiving the warning information and notifying a guardian of the warning information.

Preferably, transmitting the warning information according to the physiological state parameter of the organism includes: acquiring the physiological state parameter of the organism; performing signal conversion processing on the physiological state parameter; judging whether a value of the processed physiological state parameter exceeds a set value; and transmitting the warning information if the judging result is affirmative, and transmitting no warning information if the judging result is negative.

Preferably, geographic position of the organism is also transmitted while transmitting the warning information.

Preferably, transmitting the warning information according to the physiological state parameter of the organism further includes: manually controlling the acquiring of the physiological state parameter; and manually controlling the transmitting of the warning information.

Preferably, the physiological state parameter of the organism is also transmitted while transmitting the warning information.

Preferably, receiving the warning information and notifying the guardian of the warning information includes: receiving the warning information; performing signal conversion processing on the warning information; and notifying the guardian of the processed warning information.

Preferably, before the step of receiving the warning information, the warning method further includes: judging whether communication mode of a transmitting end is matched with that of a receiving end; and receiving the warning information if the judging result is affirmative, and receiving no warning information if the judging result is negative.

The present invention has the following beneficial effects: as the warning device provided by the present invention is provided with the initial unit and the terminal unit, when an physical abnormal condition (such as heart attack, hypertension attack, loss of consciousness after sudden falling down, etc.) occurs on the organism carrying the initial unit, warning information can be timely sent to the terminal unit, so that the terminal unit timely notifies the organism's guardian to deal with the physical abnormal condition of the organism in time so as not to cause delay in treatment.

In the warning method provided by the present invention, through transmitting the warning information according to the physiological state parameter of the organism, and receiving the warning information and notifying the guardian of the warning information, warning can be timely sent to the guardian with respect to the physiological state parameter of the organism, such that measures on the abnormal condition of the organism can be took in time, thus grasping precious time for rescue so as not to cause delay in treatment.

### Brief Description of the Drawings

Fig. 1 is a principle block diagram of a warning device in embodiment 1 of the present invention;
Fig. 2 is a flow diagram of a warning method in embodiment 1 of the present invention;
Fig. 3 is a sub-flow diagram of step 101 in Fig. 2;
Fig. 4 is a sub-flow diagram of step 102 in Fig. 2;
Fig. 5 is a principle block diagram of a warning device in embodiment 2 of the present invention.

### Reference numerals:

1. initial unit; 11. acquisition module; 12. first processing module;
13. judgment module; 14. transmitting module;
15. GPS positioning module; 16. input module; 17. first storage module;
18. first control module; 2. terminal unit; 21. receiving module;
22. second processing module; 23. notifying module;
24. second storage module; 25. second control module.

### Detailed Description of the Embodiments

To make a person skilled in the art better understand the technical solution of the present invention, a warning device and a warning method provided by the present invention are further described below in details in conjunction with the accompanying drawings and the specific embodiments.

### Embodiment 1:

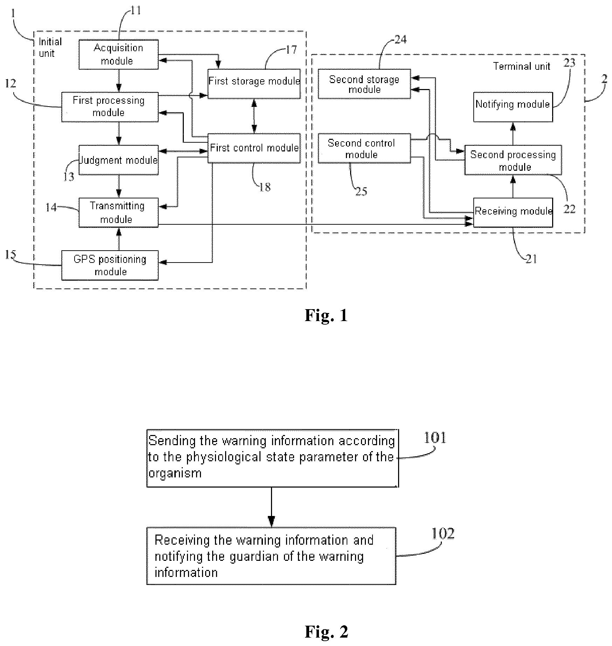

The embodiment provides a warning device, as shown in Fig. 1, including an initial unit 1 and a terminal unit 2, wherein the initial unit 1 is capable of being carried on an organism, and configured to acquire a physiological state parameter of the organism and transmit warning information according to the physiological state parameter; and the terminal unit 2 is configured to receive the warning information, and notify a guardian of the warning information.

The organism includes a human body and an animal body. For a creature individual, the warning device is provided with the initial unit 1 and the terminal unit 2, wherein when a physical abnormal condition (such as heart attack, hypertension attack, loss of consciousness after sudden falling down, etc.) occurs to the individual, the initial unit 1 carried on the individual can timely transmit warning information to the terminal unit 2, so that the terminal unit 2 timely notifies the individual's guardian to deal with the physical abnormal condition of the individual in time so as not to cause delay in treatment.

In the embodiment, the initial unit 1 includes an acquisition module 11, a first processing module 12, a judgment module 13 and a transmitting module 14. The acquisition module 11 is configured to acquire the physiological state parameter of the organism, and transmit the physiological state parameter to the first processing module 12; the first processing module 12 is configured to perform signal conversion processing on the physiological state parameter, and transmit the processed physiological state parameter to the judgment module 13; the judgment module 13 is configured to judge whether a value of the physiological state parameter exceeds a set value, and transmit a judgment result to the transmitting module 14; and the transmitting module 14 is configured to transmit the warning information to the terminal unit 2 according to the judgment result.

The physiological state parameter includes a blood pressure parameter, a pulse parameter, a heart rate parameter and an organism state parameter (such as an organism state curve, i.e. the state curve when the organism stands normally being different from that when the organism falls down suddenly), etc. The acquisition module 11 includes a blood pressure sensor, a pulse sensor, a heart rate sensor, an acceleration sensor, a gyroscope, etc., and may also include one or more of them. That is, the acquisition module 11 may acquire the blood pressure parameter, the pulse parameter, the heart rate parameter, etc. of the organism, to know whether the physiological state of the organism is abnormal, or acquire the organism state parameter (such as organism attitude, i.e., standing or falling down, etc) by the acceleration sensor or the gyroscope, to know whether abnormal change has occurred in the physical condition of the organism.

The physiological state parameter acquired by the acquisition module 11 is usually an electric signal (such as current or voltage signal) or a signal in other form, and cannot be directly compared with the set value, so the electric signal or signal in other form needs signal conversion processing by the first processing module 12, to be converted to a signal of the same type as that of the set value, to allow comparison therebetween.

Generally, a normal threshold of the physiological state parameter of the organism may be stored in the warning device in advance. Through judgment by the judgment module 13, once an abnormal condition that the value of the physiological state parameter of the organism exceeds the normal threshold occurs, the transmitting module 14 is immediately enabled to transmit the warning information. Providing the judgment module 13 also allows the warning device to automatically detect which physiological state of the organism was abnormal, so as to be beneficial for the guardian to subsequently timely cope with the abnormal physiological state; furthermore, when abnormality occurs, a human will sometimes also lose consciousness, so providing the judgment module 13 greatly facilitate automatically and timely transmitting the warning information to the guardian.

In the embodiment, preferably, the initial unit 1 further includes a first storage module 17 and a first control module 18. The first storage module 17 is connected with the first control module 18, the acquisition module 11 and the first processing module 12 respectively, and configured to store information on the physiological state parameter acquired by the acquisition module 11 and the physiological state parameter processed by the first processing module 12 under the control of the first control module 18. The first control module 18 is connected with the acquisition module 11, the first processing module 12, the judgment module 13 and the transmitting module 14 respectively, and used to control operation of the modules. Preferably, the first control module 18 is configured to control the on and off of the transmitting module 14 according to the judgment result of the judgment module 13. Furthermore, preferably, if necessary, the first control module 18 may further control that the information on the physiological state parameter stored in the first storage module 17 is read out to the transmitting module 14, which in turn transmits the information on the physiological state parameter of the organism to the receiving module 21 of the terminal unit 2.

In the embodiment, the terminal unit 2 includes a receiving module 21, a second processing module 22 and a notifying module 23; the receiving module 21 is configured to receive the warning information from the transmitting module 14 of the initial unit 1, and transmit the warning information to the second processing module 22; the second processing module 22 is configured to perform signal conversion processing on the warning information, and transmit the processed warning information to the notifying module 23; and the notifying module 23 is configured to notify the guardian of the warning information.

As the signal form of the warning information received by the receiving module 21 is different from the form of a signal that can be received and/or notified by the notifying module 23, the notifying module 23 cannot directly receive the warning information, which needs signal conversion processing by the second processing module 22 so as to be received and notified by the notifying module 23.

Preferably, the terminal unit 2 further includes a second storage module 24 and a second control module 25 that are connected to each other; the second storage module 24 is further connected with the receiving module 21 and the second processing module 22 respectively, and configured to store the warning information received by the receiving module 21 and the processed warning information processed by the second processing module 22, under the control of the second control module 25. Furthermore, preferably, the receiving module 21 of the terminal unit 2 may also receive the information on the physiological state parameter of the organism from the transmitting module 14 of the initial unit 1, and the second processing module 22 may also process the information into a signal in the same form as the signal that can be received and/or notified by the notifying module 23, so as to notify the guardian of the warning information together with the physiological state parameter of the organism at that time by the notifying module 23. The preferred solution is beneficial for the guardian to know the specific parameter of abnormal physiological state of the organism when the initial unit 1 transmits the warning information, to facilitate the guardian timely and reasonably preparing for a rescue plan.

The first storage module 17, the first control module 18, the second storage module 24 and the second control module 25 are all arranged in a central processing unit (CPU) of the warning device. Providing the first storage module 17, the first control module 18, the second storage module 24 and the second control module 25 makes the warning device more intelligent.

In the embodiment, the initial unit 1 further includes a GPS positioning module 15; the GPS positioning module 15 is connected with the transmitting module 14 and the first control module 18, and configured to locate the geographic position of the organism; the first control module 18 is configured to control whether the GPS positioning module 15 transmits the geographic position to the transmitting module 14 according to the judgment result of the judgment module 13; and the transmitting module 14 is further configured to transmit the geographic position sent by the GPS positioning module 15 to the terminal unit 2. Providing the GPS positioning module 15 allows the terminal unit 2 to also receive the geographic position of the organism transmitting the warning information while receiving the warning information, making it more convenient to timely rescue the individual with body abnormality.

The notifying module 23 includes a speaker, a vibrator and a display or any combination thereof. That is, the notifying module 23 notifies the guardian by making a sound or vibration or displaying the warning information, so that the guardian knows the emergency situation. The specific implementation of the notifying module 23 may be selected flexibly, so that the warning device is more powerful in function and more convenient to use.

In the embodiment, the transmitting module 14 and the receiving module 21 are matched with each other in transmission mode or communication mode. For example, the transmitting module 14 and the receiving module 21 both adopt a radio transmission mode, and of course may also adopt other wireless transmission mode. In the embodiment, the transmitting module 14 and the receiving module 21 adopt a radio transmission mode, and the transmitting module 14 and the receiving module 21 are also provided with matched identification codes. The transmitting module 14 and the receiving module 21 can communicate normally with each other (i.e. transmitting and receiving the warning information) only when their identification codes are matched. This facilitates point-to-point matching use of the transmitting module 14 and the receiving module 21.

Of course, the transmitting module 14 and the receiving module 21 may also adopt a same communication frequency (i.e., the transmitting module 14 transmits a signal at a frequency, and the receiving module 21 receive a signal at the same frequency), such that the matched identification codes are not needed.

The warning device in the embodiment further includes an ring band (not shown in Fig. 1), wherein the initial unit 1 is arranged on the ring band, and the ring band is wearable on the wrist, ankle or neck of the organism. On the other hand, the terminal unit 2 can be carried by the guardian or arranged at a fixed position. Preferably, the terminal unit 2 can also be arranged on another ring band, so that the terminal unit 2 can be carried on the body of the guardian. The ring bands greatly facilitate carrying the warning device, and also greatly facilitate transmitting and receiving the warning information.

Furthermore, when arranged at a fixed position, the terminal unit 2 can timely transmit the warning information to the individual's guardian while receiving the warning information. For example, the terminal unit 2 is a computer or information transmitting station, and the computer or information transmitting station can notify the guardian by an email, micro blog, short message or broadcast.

Based on the above warning device, the present invention also provides a warning method, as shown in Fig. 2, including: step 101, transmitting warning information according to a physiological state parameter of an organism; and step 102, receiving the warning information and notifying a guardian of the warning information.

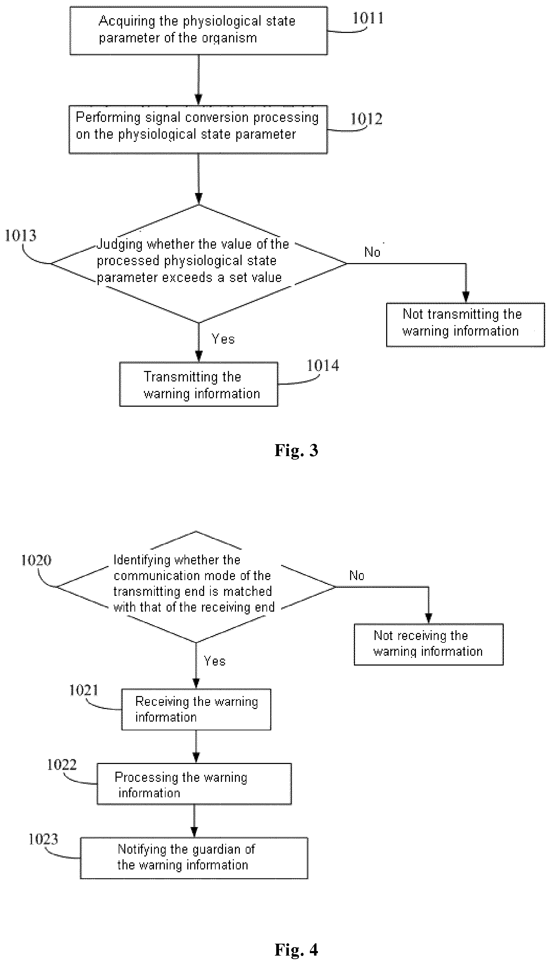

As shown in Fig. 3, step 101 of transmitting the warning information according to the physiological state parameter of the organism includes: step 1011, acquiring the physiological state parameter of the organism; step 1012, performing signal conversion processing on the physiological state parameter; step 1013, judging whether the value of the processed physiological state parameter exceeds a set value; if the judgment in step 1013 is "yes", executing step 1014 of transmitting the warning information; and if the judgment in step 1013 is "not", not transmitting the warning information.

In the warning method, whether abnormality occurs on the organism can be detected automatically, and the warning information is transmitted when abnormality occurs, to achieve timely rescue of the abnormal individual. Especially when abnormality occurs, the organism will sometimes also lose consciousness, so the warning method is very helpful to subsequently automatically and timely transmitting the warning information.

In the warning method, geographic position information of the organism is also transmitted while the warning information is transmitted, so that the guardian can timely know the geographic position of the organism to facilitate timely rescue. The geographic position of the organism may be located by the GPS positioning module.

As shown in Fig. 4, step 102 of receiving the warning information and notifying the guardian of the warning information includes: step 1021, receiving the warning information; step 1022, performing signal conversion processing on the warning information; and step 1023, notifying the guardian of the processed warning information.

Furthermore, as shown in Fig. 4, before step 1021 of receiving the warning information, the method may further include: step 1020, judging whether the communication mode of a transmitting end is matched with that of a receiving end; if the judgment in step 1020 is "yes", executing step 1021 of receiving the warning information; and if the judgment in step 1020 is "not", not receiving the warning information.

### Embodiment 2:

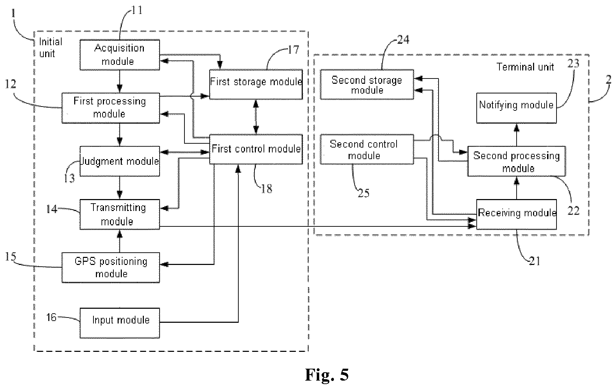

The embodiment provides a warning device, which is different from embodiment 1 in that, as shown in Fig. 5, on the basis of embodiment 1, the initial unit 1 of the warning device further includes an input module 16; the input module 16 is connected with the first control module 18, and used for manually inputting a control instruction to manually control on and off of the acquisition module 11 and manually control on and off of the transmitting module 14 through the first control module, thus achieving manual control of acquiring the physiological state parameter of the organism and transmitting the warning information to the guardian.

The input module 16 includes a microphone, a tap sensing key, a touch screen and/or a key.

Providing the input module 16 allows the organism to actively transmit the warning information to the guardian when feeling uncomfortable, so that the guardian can adopt coping measures in good time so as not to cause delay in treatment. From a certain perspective, providing the input module 16 is compensation for the function of the judgment module 13, because the judgment module 13 generally causes the transmitting module 14 to transmit the warning information only when the value of the physiological state parameter of the organism exceeds the set value, which is generally close to a risk value, or is the risk value. The input module 16 can be used actively to cause the transmitting module 14 to transmit the warning information when the organism just feels uncomfortable, so providing the input module 16 can make the warning more advanced so as not to cause delay in treatment.

Other structures of the warning device in the embodiment are the same as those in embodiment 1, and are not repeated herein.

The operation process of warning by using the input module 16 is described below specifically by taking an example: if feeling uncomfortable, the organism may touch the tap sensing key or press key on the input module 16 to instruct the first control module 18 to enable the acquisition module 11, so that the acquisition module 11 performs acquisition operation to acquire the physiological state parameter of the organism, and the organism speaks or makes other sound through the microphone on the input module 16, so that the input module 16 acquires voice information of the organism; after completion of speaking, the organism touches the tap sensing key or press key again to instruct the first control module 18 to store the voice information acquired by the input module 16 and the physiological state parameter acquired by the acquisition module 11 into the first storage module 17; and finally, the first control module 18 enables the transmitting module 14 to transmit the voice information and the physiological state parameter in the first storage module 17 together with the identification code of the transmitting module 14. The terminal unit 2 receives the identification code, and judges whether the identification code is matched with that of the receiving module 21; if they are matched, then receives the voice information and the physiological state parameter, and performs signal conversion processing on the voice information and the physiological state parameter; and finally plays back the processed voice information though the speaker, and/or displays the physiological state parameter through the display, so that the guardian carrying the terminal unit 2 can hear voice warning from the organism, and/or can observe the physiological state parameter of the organism at that time. Whether the tap sensing key or press key is touched can be indicated by on and off of an indicator lamp. If the indicator lamp is lit up, it indicates that the tap sensing key or press key is touched; and if the indicator lamp is not lit up, it indicates that the tap sensing key or press key is not touched.

It needs to be noted additionally that processing on the voice information in the initial unit 1 includes that, for example, the voice information enters a voltage controlled oscillator through an amplification circuit, a pre-emphasis circuit and a band-pass filter, then enters a modulation circuit for modulation, and is finally transmitted through an antenna after a series of radio frequency processing. Processing on the received voice information in the terminal unit 2 includes that, for example, the voice information passes through an audio signal processing circuit, and enters a volume control circuit and a power amplification circuit for processing, and then the speaker is driven to play the voice information. The specific processing process is not described herein in details.

Based on the warning device described in the embodiment, the embodiment also provides a warning method. On the basis of the warning method in embodiment 1, transmitting warning information according to the physiological state parameter of the organism in the warning method further includes: manually controlling whether to acquire the physiological state parameter; and manually controlling whether to transmit the warning information.

Other steps of the warning method in the embodiment are the same as those in embodiment 1, and are not described herein.

### Embodiment 3:

The embodiment provides a warning device, which is different from embodiments 1-2 in that, the warning device in the embodiment includes an input module, and does not include a judgment module.

Other structures of the warning device in the embodiment are the same as those in any of embodiments 1-2, and are not repeated herein. That is, the warning device provided by the embodiment can only implement manual control of transmitting the warning information, but cannot implement automatically transmitting the warning information. In spite of lacking the function of automatic warning, warning function can still be achieved in that the organism can actively transmit warning to the guardian.

Embodiments 1-3 have the following beneficial effects: as the warning device provided in embodiments 1-3 is provided with the initial unit and the terminal unit, when a physical abnormal condition (such as heart attack, hypertension attack, loss of consciousness after sudden falling down, etc.) occurs on the organism carrying the initial unit, warning information can be timely sent to the terminal unit, so that the terminal unit timely notifies the organism's guardian to deal with the physical abnormal condition of the organism in time so as not to cause delay in treatment.

It should be understood that the above embodiments are only exemplary embodiments for illustrating the principle of the present invention, however, the present invention is not limited thereto. Various modifications and improvements can be made by the person skilled in the art without departing from the spirit and essence of the present invention, and these modifications and improvements should also be considered to be within the protection scope of the present invention.

## Claims

1. A warning device, comprising an initial unit and a terminal unit, wherein the initial unit is capable of being carried on an organism, and configured to acquire a physiological state parameter of the organism and transmit warning information according to the physiological state parameter; and wherein the terminal unit is configured to receive the warning information, and notify a guardian of the warning information.

2. The warning device according to claim 1, wherein the initial unit comprises an acquisition module, a first processing module, a judgment module and a transmitting module; the acquisition module is configured to acquire the physiological state parameter of the organism, and transmit the physiological state parameter to the first processing module; the first processing module is configured to perform signal conversion processing on the physiological state parameter, and transmit the processed physiological state parameter to the judgment module; the judgment module is configured to judge whether a value of the processed physiological state parameter exceeds a set value, and transmit a judgment result to the transmitting module; and the transmitting module is configured to transmit the warning information to the terminal unit according to the judgment result.

3. The warning device according to claim 2, wherein the terminal unit comprises a receiving module, a second processing module and a notifying module; the receiving module is configured to receive the warning information, and transmit the warning information to the second processing module; the second processing module is configured to perform signal conversion processing on the warning information, and transmit the processed warning information to the notifying module; and the notifying module is configured to notify the guardian of the warning information.

4. The warning device according to claim 3, wherein the initial unit further comprises a first storage module and a first control module that are connected to each other; the first storage module is further connected with the acquisition module and the first processing module respectively, and configured to store the physiological state parameter acquired by the acquisition module and the processed physiological state parameter processed by the first processing module, under the control of the first control module;
the first control module is further connected with the judgment module and the transmitting module respectively, and configured to control on and off of the transmitting module according to the judgment result of the judgment module; and
the first control module further controls that the physiological state parameter stored in the first storage module is read out to the transmitting module, which in turn transmits the physiological state parameter of the organism to the terminal unit.

5. The warning device according to claim 4, wherein the terminal unit further comprises a second storage module and a second control module that are connected to each other; the second storage module is further connected with the receiving module and the second processing module respectively, and configured to store the warning information received by the receiving module and the processed warning information processed by the second processing module, under the control of the second control module;
the receiving module of the terminal unit is further configured to receive the physiological state parameter of the organism from the transmitting module of the initial unit, and transmit the physiological state parameter to the second processing module; the second processing module is further configured to perform signal conversion processing on the physiological state parameter, and transmit the processed physiological state parameter to the notifying module; and the notifying module is further configured to notify the guardian of the physiological state parameter.

6. The warning device according to claim 4, wherein the initial unit further comprises a GPS positioning module; the GPS positioning module is connected with the transmitting module and the first control module, and configured to locate the geographic position of the organism; the first control module is configured to control the GPS positioning module transmitting the geographic position to the transmitting module according to the judgment result of the judgment module; and the transmitting module is further configured to transmit the geographic position to the terminal unit.

7. The warning device according to claim 4, wherein the initial unit further comprises an input module; the input module is connected with the first control module, and configured to receive a manually input control signal so as to manually control the on and off of the acquisition module and manually control the on and off of the transmitting module.

8. The warning device according to claim 7, wherein the input module comprises a microphone, a tap sensing key, a touch screen or a press key; and the notifying module comprises a speaker, a vibrator and/or a display.

9. The warning device according to claim 2, wherein the acquisition module comprises a blood pressure sensor, a pulse sensor, a heart rate sensor, an acceleration sensor and/or a gyroscope.

10. The warning device according to claim 4, wherein the transmitting module and the receiving module are matched with each other in transmission mode or communication mode.

11. The warning device according to any of claims 1-10, further comprising a ring band, wherein the initial unit is arranged on the ring band which is wearable on the wrist, ankle or neck of the organism; and wherein the terminal unit is capable of being carried by the guardian or arranged at a fixed position.

12. A warning method, comprising: transmitting warning information according to a physiological state parameter of an organism; and receiving the warning information and notifying a guardian of the warning information.

13. The warning method according to claim 12, wherein the transmitting of the warning information according to the physiological state parameter of the organism comprises: acquiring the physiological state parameter of the organism; performing signal conversion processing on the physiological state parameter; judging whether a value of the processed physiological state parameter exceeds a set value; and transmitting the warning information if the judging result is affirmative, and transmitting no warning information if the judging result is negative.

14. The warning method according to claim 13, wherein geographic position of the organism is also transmitted while transmitting the warning information.

15. The warning method according to claim 14, wherein the transmitting of the warning information according to the physiological state parameter of the organism further comprises: manually controlling the acquiring of the physiological state parameter; and manually controlling the transmitting of the warning information.

16. The warning method according to claim 15, wherein the physiological state parameter of the organism is also transmitted while transmitting the warning information.

17. The warning method according to claim 15, wherein the receiving of the warning information and notifying the guardian of the warning information comprises: receiving the warning information; performing signal conversion processing on the warning information; and notifying the guardian of the processed warning information.

18. The warning method according to any of claims 12-17, wherein prior to the transmitting and receiving of the warning information, the method further comprises: judging whether communication mode of a transmitting end is matched with that of a receiving end; and receiving the warning information if the judging result is affirmative, and receiving no warning information if the judging result is negative.
